# EUROPEAN PATENT APPLICATION

(11) **EP 4 446 311 A1**
(43) Date of publication of application: **16.10.2024**
(21) Application number: 22903156.2
(22) Date of filing: 17.11.2022
(51) Int. Cl.: C07C 271/44, C07C 47/277, C07C 219/04, C07C 229/12, A61P 25/04, A61K 31/27, A61P 29/00, A61K 31/265, A61P 25/08

(54) **CANNABIDIOL DERIVATIVE, AND PREPARATION METHOD THEREFOR AND USE THEREOF**

(30) Priority: 10.12.2021 CN 202111516427
(71) Applicant: Deyi Pharmaceutical Ltd., Kunming, Yunnan 650100 (CN)
(72) Inventor: WANG, Shubin, Kunming, Yunnan 650100 (CN); DU, Yesong, Kunming, Yunnan 650100 (CN); ZHANG, Pingping, Kunming, Yunnan 650100 (CN)
(74) Representative: HGF
(86) International application number: PCT/CN2022/132572
(87) International publication number: WO 2023/103733

(57) **Abstract**

Disclosed are a cannabidiol derivative, and a preparation method therefor and the use thereof, and in particular the use thereof in the prevention and treatment of a nervous system disease (such as epilepsy and Parkinson's disease). The cannabidiol derivative is obtained by means of screening from a series of synthetic derivatives, and animal test results show that the compounds can effectively shorten the epileptic seizure duration of an experimental animal, improves the epileptic seizure symptoms of the experimental animal, reduces the balance beam score of the Parkinson's model animal, increases the dopamine level and the tyrosine hydroxylase (TH) cell positivity in the substantia nigra (SubN), can be used for drug development and research on various diseases such as epilepsy and Parkinson's disease, and has a better application value.

## Description

### TECHNICAL FIELD

The present invention relates to the technical field of chemical pharmaceuticals, and in particular to a cannabidiol derivative, a preparation method therefor, and use thereof, particularly use thereof in the prevention and treatment of a nervous system disease (e.g., epilepsy or Parkinson's disease).

### BACKGROUND

Cannabinoids are a class of secondary metabolites containing alkyl and monoterpene group structures that are unique to cannabis plants. To date, more than a hundred cannabinoids have been isolated from cannabis plants, mainly including δ-9-tetrahydrocannabinol (THC), cannabidiol (CBD), cannabichromene (CBC), cannabigerol (CBG), cannabinol (CBN), etc., with THC and CBD having the highest content. Studies have shown that cannabinoids have a wide range of pharmacological effects.

Cannabidiol (CBD), with the chemical name of 2-[(1*R*,6*R*)-3-methyl-6-(1-methylvinyl)-2-cyclohexen-1-yl]-5-pentyl-1,3-benzenediol and CAS registry number of 13956-29-1, has a chemical structure shown as follows:

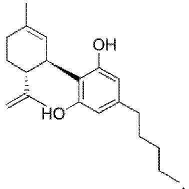

Studies have found that CBD has pharmacological effects of spasm resistance, anxiety resistance, inflammation resistance, oxidation resistance, and the like, and can be used for drug development. However, high-purity CBD is a white or pale yellow crystal with a melting point of 66-67 °C, and is almost insoluble in water or 10% sodium hydroxide solution, and easily soluble in organic solvents such as ethanol, methanol, diethyl ether, benzene, chloroform, and petroleum ether. CBD has poor water solubility and low bioavailability, which greatly limits the application of CBD in the fields of pharmaceuticals and the like.

### SUMMARY

In order to overcome the defects of the prior art, the present invention provides a cannabidiol derivative, a preparation method therefor, and use thereof, particularly use thereof in the prevention and treatment of a nervous system disease (e.g., epilepsy or Parkinson's disease).

In a first aspect, the present invention provides a compound having the following structure: 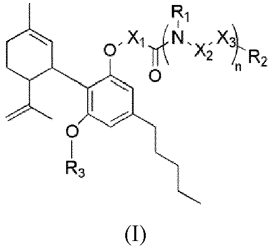 wherein,
X₁ and X₂ are independently selected from: a single bond, substituted or unsubstituted alkylene, alkylene spaced by one or more -C(=O)O-, alkylene spaced by one or more -OC(=O)-, and alkylene spaced by one or more -C(=O)NH-;
X₃ is selected from: 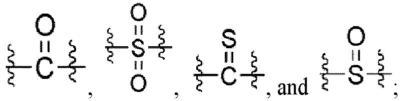
R₁ is selected from: H, alkyl, and cycloalkyl;
R₂ is selected from: OH and alkoxy;
n is an integer of 1-5 (e.g., 1, 2, 3, 4, or 5);
R₃ is selected from: Hand 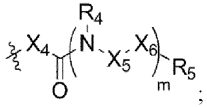
wherein, X₄ and X₅ are independently selected from: a single bond, substituted or unsubstituted alkylene, alkylene spaced by one or more -C(=O)O-, alkylene spaced by one or more -OC(=O)-, and alkylene spaced by one or more -C(=O)NH-;
X₆ is selected from: 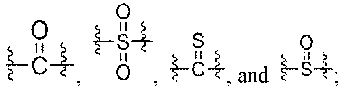
R₄ is selected from: H, alkyl, and cycloalkyl;
R₅ is selected from: OH and alkoxy;
m is an integer of 1-5 (e.g., 1, 2, 3, 4, or 5).

Specifically, X₁ is selected from: a single bond, linear or branched C1-6 alkylene, particularly linear C1-3 alkyl; in one embodiment of the present invention, X₁ is methylene.

In one embodiment of the present invention, X₂ has the following structure: 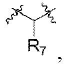 wherein R₇ is selected from: H, alkyl, alkyl substituted with alkylsulfydryl, alkyl substituted with hydroxy, alkyl substituted with sulfydryl, alkyl substituted with -C(=O)NH₂, alkyl substituted with carboxyl, alkyl substituted with amino, alkyl substituted with 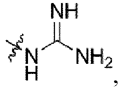 heterocyclylalkyl, and aralkyl.

Specifically, R₇ can be selected from: H, methyl, isopropyl, sec-butyl, isobutyl, 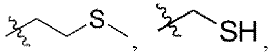 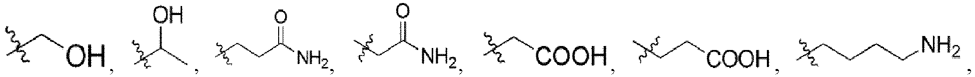 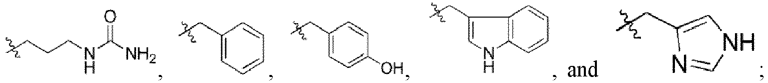 in one embodiment of the present invention, R₇ is

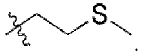

In some embodiments of the present invention, X₃ is

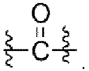

In some embodiments of the present invention, n is 1.

In some embodiments of the present invention, R₁ is H.

Specifically, R₂ is selected from: OH and C1-6 alkoxy; in some embodiments of the present invention, R₂ is OH, methoxy, or ethoxy. In one embodiment of the present invention, the compound described above has the following structure:

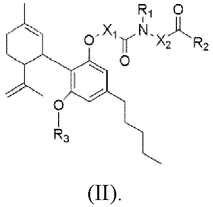

More specifically, the compound described above has the following structure:

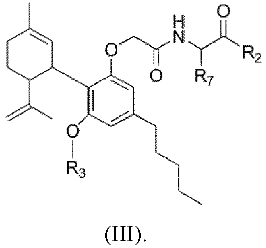

In one embodiment of the present invention, R₃ is H.

In another embodiment of the present invention, R₃ is

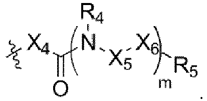

Specifically, X₄ is selected from: a single bond and linear or branched C1-6 alkylene, particularly linear C1-3 alkyl; in one embodiment of the present invention, X₄ is methylene.

In one embodiment of the present invention, X₅ has the following structure: 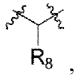 wherein R₈ is selected from: H, alkyl, alkyl substituted with alkylsulfydryl, alkyl substituted with hydroxy, alkyl substituted with sulfydryl, alkyl substituted with -C(=O)NH₂, alkyl substituted with carboxyl, alkyl substituted with amino, alkyl substituted with 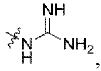 heterocyclylalkyl, and aralkyl.

Specifically, R₈ may be selected from: H, methyl, isopropyl, sec-butyl, isobutyl, 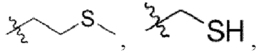 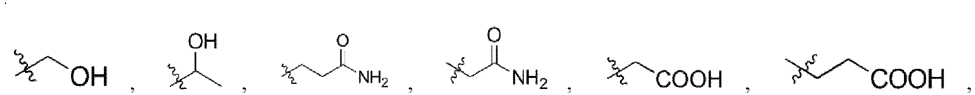 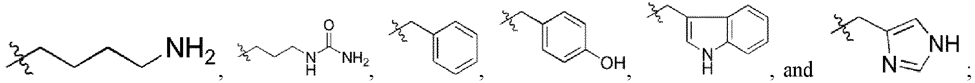 in one embodiment of the present invention, R₈ is

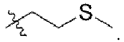

In some embodiments of the present invention, X₆ is

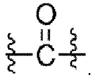

In some embodiments of the present invention, m is 1.

In some embodiments of the present invention, R₄ is H.

Specifically, R₅ is selected from: OH and C1-6 alkoxy; in some embodiments of the present invention, R₅ is OH, methoxy, or ethoxy. In one embodiment of the present invention, the compound described above has the following structure:

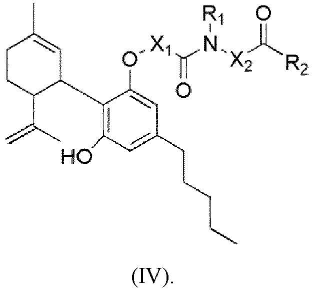

In another embodiment of the present invention, the compound described above has the following structure:

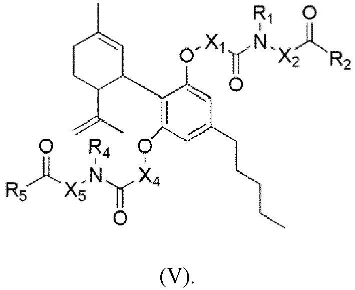

In some embodiments of the present invention, the compound described above has the following structure:

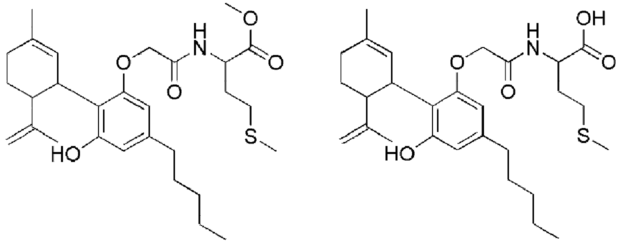

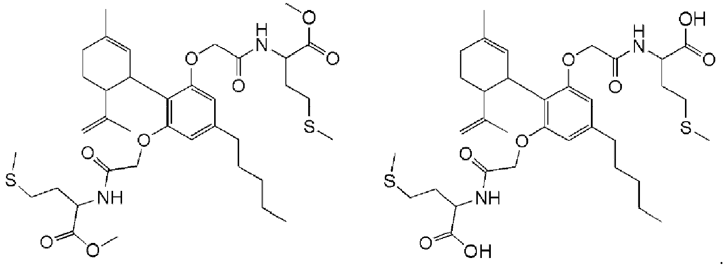

In a second aspect of the present invention, provided is a stereoisomer of the compound according to the first aspect, which has the following structure: 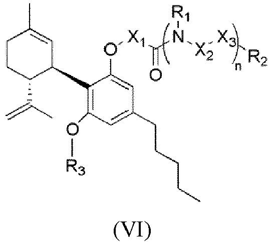 wherein, X₁, X₂, X₃, R₁, R₂, R₃, and n have corresponding definitions as described in the first aspect of the present invention.

In some embodiments of the present invention, the stereoisomer described above has the following structure:

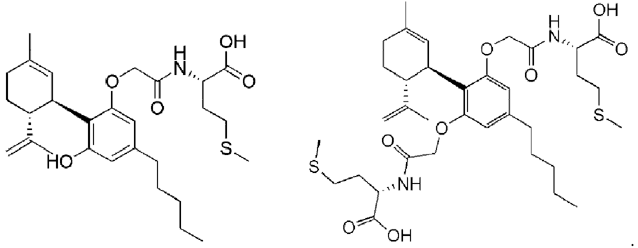

In a third aspect of the present invention, provided is a salt, particularly a pharmaceutically acceptable salt, of the compound according to the first aspect or the stereoisomer according to the second aspect.

Specifically, the salt is a base addition salt, which can be formed from a metal or amine with the compound, particularly an alkali metal salt or an alkaline earth metal salt, e.g., a sodium salt, a potassium salt, a magnesium salt, or a calcium salt.

In some embodiments of the present invention, the salt is 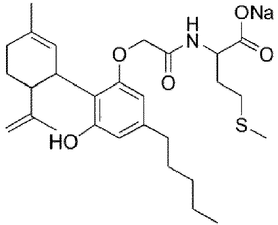 or

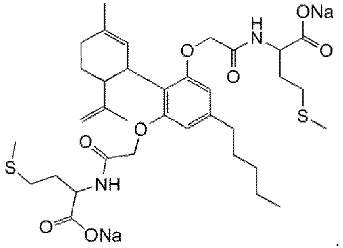

In a fourth aspect of the present invention, provided is an ester, a prodrug, or a solvate of the compound according to the first aspect.

In a fifth aspect of the present invention, provided is a preparation method for the compound according to the first aspect.

In one embodiment of the present invention, R₃ is H, and the preparation method can comprise the following reaction step: 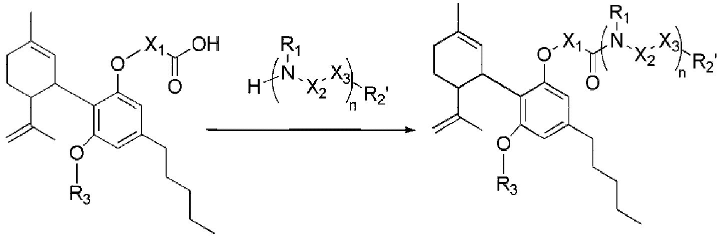 wherein, R₂' is alkoxy, e.g., C1-6 alkoxy, e.g., methoxy.

In some embodiments of the present invention, 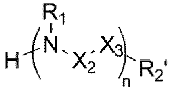 in the reaction step described above is

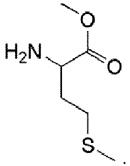

Specifically, the preparation method can also comprise the following reaction step:

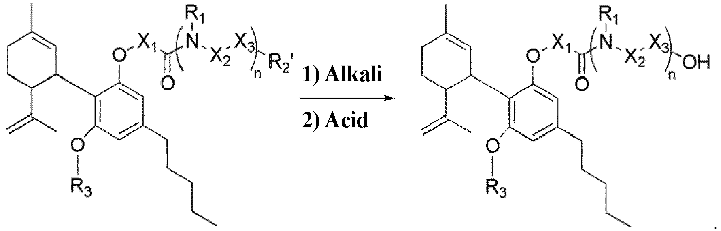

Specifically, the base in the reaction step described above is a hydroxide of an alkali metal, e.g., lithium hydroxide, sodium hydroxide, or potassium hydroxide.

Specifically, the acid in the reaction step described above is an inorganic acid, e.g., hydrochloric acid. Specifically, the preparation method can also comprise the following reaction step: 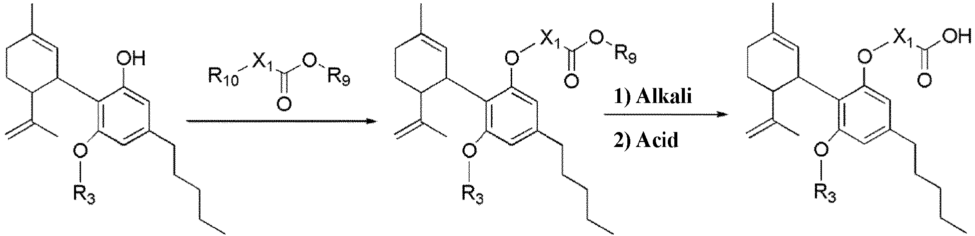
wherein, R₉ is alkyl, e.g., C1-6 alkyl, e.g., methyl;
R₁₀ is a leaving group, e.g., -F, -Cl, -Br, -I,

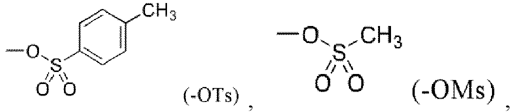

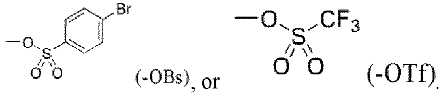

In some embodiments of the present invention, 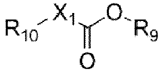 in the reaction step described above is

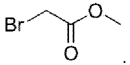

Specifically, the base in the reaction step described above is a hydroxide of an alkali metal, e.g., lithium hydroxide, sodium hydroxide, or potassium hydroxide.

Specifically, the acid in the reaction step described above is an inorganic acid, e.g., hydrochloric acid.

In another embodiment of the present invention, R₃ is 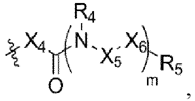 and the preparation method can comprise a step of subjecting 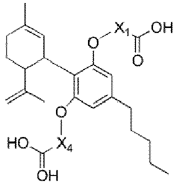 to a reaction with 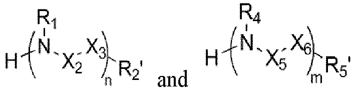 separately or simultaneously (in this case, 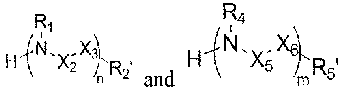 represent the same substance); wherein, R₂' and R₅' are alkoxy, e.g., C1-6 alkoxy, e.g., methoxy.

In one embodiment of the present invention, 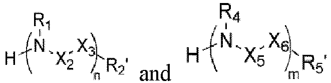 represent the same substance, e.g.,

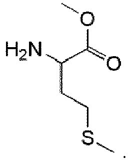

In a sixth aspect of the present invention, provided is a pharmaceutical composition, which comprises the compound according to the first aspect of the present invention, or the pharmaceutically acceptable salt, the stereoisomer, the ester, the prodrug or the solvate thereof, and one or more pharmaceutically acceptable excipients.

Specifically, the pharmaceutically acceptable excipients can be selected from: one or more of fillers, binders, lubricants, disintegrants, antioxidants, buffers, suspending agents, solubilizers, thickeners, stabilizers, preservatives, and the like.

Specifically, the pharmaceutical composition can be administered enterally or parenterally, e.g., by intravenous, intramuscular, intradermal, and subcutaneous routes.

Specifically, the pharmaceutical composition can be prepared into the following dosage forms: tablets, pills, powders, granules, capsules, lozenges, syrups, gels, emulsions, suspensions, controlled release preparations, aerosols, films, injections, intravenous drip infusions, transdermal absorption formulations, ointments, lotions, adhesive formulations, suppositories, nasal formulations, pulmonary formulations, and the like.

Specifically, various dosage forms of the pharmaceutical composition can be prepared according to conventional production methods in the pharmaceutical field.

Specifically, the pharmaceutical composition can also comprise one or more other active ingredients for the same or different indications.

In a seventh aspect of the present invention, provided is use of the compound according to the first aspect of the present invention, or the pharmaceutically acceptable salt, the stereoisomer, the ester, the prodrug or the solvate thereof, or the pharmaceutical composition according to the sixth aspect of the present invention in the preparation of a medicament for preventing and/or treating a disease.

Specifically, the disease described above is selected from: one or more of a nervous system disease, cancer, an autoimmune disease, a cardiovascular disease, pain, inflammation, and liver injury, particularly a nervous system disease.

Specifically, the nervous system disease includes, but is not limited to, epilepsy, multiple sclerosis, Parkinson's disease, Alzheimer's disease, dementia with Lewy bodies, Huntington's disease, anxiety, depression, and the like. Specifically, the epilepsy may be acute epilepsy or chronic epilepsy, particularly chronic epilepsy.

Specifically, the cancer includes, but is not limited to, breast cancer, lung cancer, colorectal cancer, liver cancer, pancreatic cancer, gastric cancer, gastroesophageal adenocarcinoma, esophageal cancer, small intestine cancer, gastric cardia cancer, endometrial cancer, ovarian cancer, fallopian tube cancer, vulval cancer, testicular cancer, prostate cancer, leukemia, glioma, and the like.

Specifically, the autoimmune disease includes, but is not limited to, systemic lupus erythematosus, rheumatoid arthritis, multiple sclerosis, ulcerative colitis, Crohn's disease, autoimmune hepatitis, and the like.

In an eighth aspect of the present invention, provided is a method for preventing and/or treating a disease, which comprises a step of administering to a subject in need thereof the compound according to the first aspect of the present invention, or the pharmaceutically acceptable salt, the stereoisomer, the ester, the prodrug or the solvate thereof, or the pharmaceutical composition according to the sixth aspect of the present invention.

Specifically, the disease described above is selected from: one or more of a nervous system disease, cancer, an autoimmune disease, a cardiovascular disease, pain, inflammation, and liver injury, particularly a nervous system disease.

Specifically, the nervous system disease includes, but is not limited to, epilepsy, multiple sclerosis, Parkinson's disease, Alzheimer's disease, dementia with Lewy bodies, Huntington's disease, anxiety, depression, and the like. Specifically, the epilepsy may be acute epilepsy or chronic epilepsy, particularly chronic epilepsy.

Specifically, the cancer includes, but is not limited to, breast cancer, lung cancer, colorectal cancer, liver cancer, pancreatic cancer, gastric cancer, gastroesophageal adenocarcinoma, esophageal cancer, small intestine cancer, gastric cardia cancer, endometrial cancer, ovarian cancer, fallopian tube cancer, vulval cancer, testicular cancer, prostate cancer, leukemia, glioma, and the like.

Specifically, the autoimmune disease includes, but is not limited to, systemic lupus erythematosus, rheumatoid arthritis, multiple sclerosis, ulcerative colitis, Crohn's disease, autoimmune hepatitis, and the like.

Specifically, the subject may be a mammal, e.g., a human, chimpanzee, monkey, dog, rabbit, mouse, etc., particularly a human.

The inventor of the present invention carries out structural modification on the basis of cannabidiol, and obtains the compounds of the present invention by screening from a series of synthetic derivatives. Animal test results show that the compounds can effectively shorten the duration of epileptic seizure of laboratory animals, improve the symptoms of epileptic seizure of the laboratory animals, reduce the balance beam score of the model animal of Parkinson's disease, and improve the dopamine level and tyrosine hydroxylase (TH) cell positive rate in substantia nigra (SubN), so that they can be used for drug development and research of various diseases such as epilepsy and Parkinson's disease, and have relatively good application value.

### BRIEF DESCRIPTION OF THE DRAWINGS

FIG. 1 shows detection results for the duration of epileptic seizure in PTZ-kindling epileptic rats before and after treatment.
FIG. 2 shows Ono's grade for behavioral seizure indexes of PTZ-kindling epileptic rats before and after treatment.
FIG. 3 shows detection results for the latency to epileptic seizure in PTZ-kindling epileptic rats before and after treatment.
FIG. 4 shows an image of PAGE gel of apoptosis-related proteins Bax and Bcl-2 in hippocampal tissues of rats.
FIG. 5 shows changes in apoptosis-related proteins Bax and Bcl-2 in hippocampal tissues of rats.
FIG. 6 shows detection results for the content of γ-aminobutyric acid in the homogenate of brain tissues of rats.
FIG. 7 shows detection results for the content of glycine in the homogenate of brain tissues of rats.
FIG. 8 shows detection results for the content of glutamic acid in the homogenate of brain tissues of rats.
FIG. 9 shows detection results for the content of aspartic acid in the homogenate of brain tissues of rats.FIG. 10 shows results for the behavioral balance beam score of the model of Parkinson's disease.
FIG. 11 shows detection results for the content of dopamine in striatum of the model of Parkinson's disease after treatment.

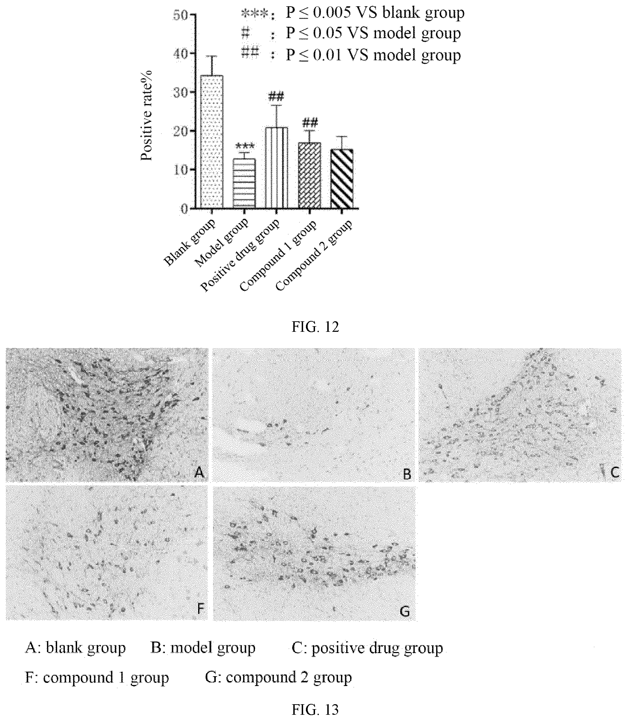

FIG. 12 shows positive rate of tyrosine hydroxylase (TH) cells in substantia nigra (SubN).
FIG. 13 shows micrographs of tyrosine hydroxylase (TH) cells in substantia nigra (SubN).

### DETAILED DESCRIPTION

Unless otherwise defined, all scientific and technical terms used in the present invention have the same meaning as commonly understood by those skilled in the art to which the present invention relates, for example:
The term "alkyl" refers to hydrocarbyl formed from an alkane molecule by losing one hydrogen atom, which may be linear or branched and is linked to other parts of the molecule by a single bond. The alkyl used herein generally contains 1 to 12 (e.g., 1, 2, 3, 4, 5, 6, 7, 8, 9, 10, 11, or 12) carbon atoms, preferably 1 to 6 carbon atoms (i.e., C₁-C₆ alkyl). Examples of the alkyl include, but are not limited to, methyl, ethyl, *n*-propyl, isopropyl, *n*-butyl, isobutyl, *tert-*butyl, *n*-pentyl, isopentyl, neopentyl, *tert*-pentyl, *n*-hexyl, isohexyl, and the like. If the alkyl is substituted with aryl, it is correspondingly "aralkyl" (e.g., C₇-C₁₈ aralkyl, e.g., C₇-C₁₅ aralkyl or C₇-C₁₂ aralkyl; e.g., (C₁-C₆ alkylene)-(C₆-C₁₂ aryl) or (C₁-C₃ alkylene)-phenyl), such as benzyl, benzhydryl, or phenethyl. If the alkyl is substituted with heterocyclyl, it is correspondingly "heterocyclylalkyl".

The term "alkylene" refers to hydrocarbyl (divalent alkyl) formed from an alkane molecule by losing two hydrogen atoms, which may be linear or branched and is linked to other parts of the molecule by a single bond. Typical alkylene herein contains 1 to 12 (e.g., 1, 2, 3, 4, 5, 6, 7, 8, 9, 10, 11, or 12) carbon atoms, preferably 1 to 6 carbon atoms. Examples of the alkylene include methylene (-CH₂-), ethylidene, propylidene, butylidene, and the like. The term "alkoxy" refers to a substituent formed from a hydroxy group by substituting the hydrogen atom with alkyl. The alkoxy used herein generally contains 1 to 12 (e.g., 1, 2, 3, 4, 5, 6, 7, 8, 9, 10, 11, or 12) carbon atoms, preferably 1 to 6 carbon atoms (i.e., Ci-Ce alkoxy). Examples of the alkoxy include, but are not limited to, methoxy, ethoxy, propoxy, butoxy, and the like.

The term "cycloalkyl" refers to an alicyclic hydrocarbon, such as those containing 1 to 4 monocyclic and/or fused rings and having 3 to 18 carbon atoms, preferably 3 to 10 (e.g., 3, 4, 5, 6, 7, 8, 9, or 10) carbon atoms, e.g., cyclopropyl, cyclobutyl, cyclopentyl, cyclohexyl, adamantyl, or the like.

The term "aryl" refers to any functional group or substituent derived from a simple aromatic ring, including monocyclic aryl groups and/or fused aryl groups, such as those containing 1 to 3 monocyclic or fused rings, and having 6 to 18 (e.g., 6, 8, 10, 12, 14, 16, or 18) carbon ring atoms. The aryl used herein is generally an aryl group containing 1 to 2 monocyclic or fused rings and having 6-12 carbon ring atoms (i.e., C₆-C₁₂ aryl), wherein H on the carbon atoms may be substituted, for example, with alkyl, halogen, and the like. Examples of the aryl include, but are not limited to, phenyl, p-hydroxyphenyl, and the like.

The term "heterocyclyl" refers to a 3- to 18-membered non-aromatic ring group containing 2 to 17 carbon atoms and 1 to 10 heteroatoms. The heterocyclyl may be a monocyclic, bicyclic, tricyclic or tetracyclic ring system, which may include fused, spiro or bridged ring systems. The heterocyclyl may be partially saturated (heteroaryl) or fully saturated (heterocycloalkyl).

The term "pharmaceutically acceptable salt" includes both acid addition salts and base addition salts.

The term "stereoisomer" includes enantiomeric, diastereomeric, and geometric isomer forms.

The term "solvate" refers to a physical association of the compound of the present invention with one or more solvent molecules. The physical association includes various degrees of ionic and covalent bonding, including hydrogen bonding. In some cases, the solvate can be isolated, for example, when one or more solvent molecules are incorporated into the crystal lattice of a crystalline solid. Solvates include both solution phases and isolatable solvates. Representative solvates include ethanolates, methanolates, and the like.

The term "prodrug" refers to forms of the compound of formula I (including acetals, esters, and zwitterions) which are suitable for administration to patients without undue toxicity, irritation, allergic response and the like, and which are effective for the intended use thereof. The prodrug is converted *in vivo,* e.g., by hydrolysis in blood, to give the parent compound.

The term "subject" refers to any animal or cell thereof, whether *in vitro* or *in situ,* that receives the methods described herein. Specifically, the animal described above includes mammals, e.g., rats, mice, guinea pigs, rabbits, dogs, monkeys, chimpanzees, or humans, particularly humans.

The term "treating" refers to preventing, curing, reversing, attenuating, alleviating, minimizing, suppressing, arresting, and/or stopping one or more clinical symptoms of a disease after its onset.

The term "preventing" refers to treatment to avoid, minimize, or make difficult the onset or progression of a disease prior to its onset.

The disclosures of the various publications, patents, and published patent specifications cited herein are hereby incorporated by reference in their entireties.

The technical solutions of the present invention will be clearly and completely described below with reference to the examples of the present invention, and it is obvious that the described examples are only a part of the examples of the present invention but not all of them. Based on the examples of the present invention, all other examples obtained by those of ordinary skills in the art without creative work shall fall within the protection scope of the present invention.

### Example 1: Preparation of Compound E1

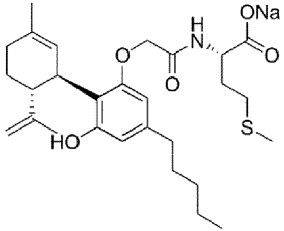

### 1. Synthetic route

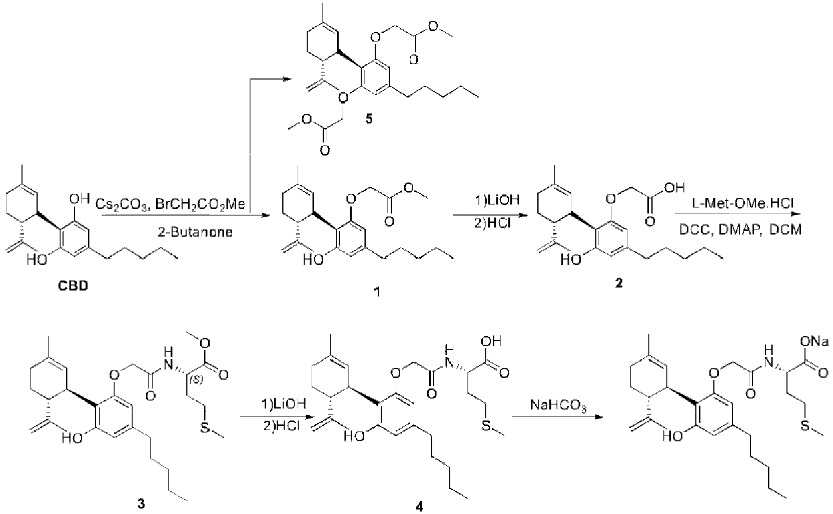

### 2. Synthetic method

### (1) Synthesis of compound 2

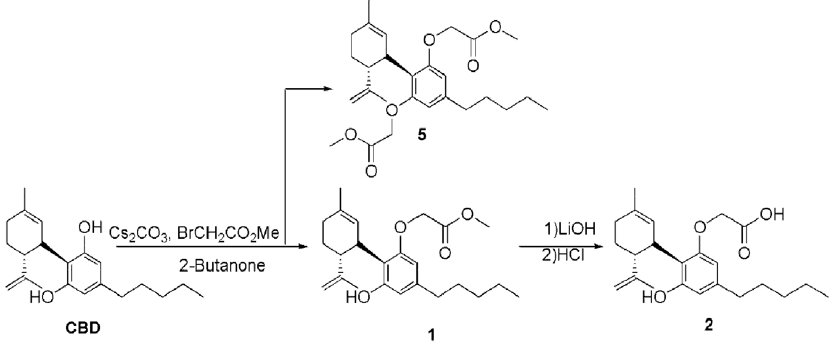

### Experimental procedures:

CBD (20 g, 64 mmol, 1 eq) was dissolved in 400 mL of butanone, cesium carbonate (31 g, 96 mmol, 1.5 eq) was added, and the mixture was heated to 80 °C and reacted for 2 h. Methyl bromoacetate 12.6 g (0.082 mol, 1.3 eq) was added, and the mixture was reacted overnight. Methyl bromoacetate (5 g) and cesium carbonate (15 g) were added, and the mixture was reacted for 2 h. The reaction solution was filtered. After concentration, ethyl acetate (100 mL) was added, and the mixture was washed sequentially with 2 N hydrochloric acid and saturated sodium chloride. The organic phase was collected, dried over anhydrous sodium sulfate, and concentrated to give a crude product (30 g) as a brown oil. The crude product was subjected to column chromatography (n-hexane/dichloromethane 20:1-1:1) to give compound **1** (11 g) and compound 5 (10.5 g) as yellow oils.

Compound **1** (11 g, 28.5 mmol, 1 eq) was dissolved in THF (100 mL), and lithium hydroxide (3.6 g, 85 mmol, 3 eq) was dissolved in 15 mL of water. The above solutions were mixed and stirred overnight.

The reaction solution was adjusted to pH 3-4 with 2 N dilute hydrochloric acid. Ethyl acetate (300 mL) was added, and the mixture was subjected to liquid separation. The organic phase was washed with saturated sodium chloride, dried over anhydrous sodium sulfate, filtered, and concentrated to give compound **2** (9.5 g) as a yellow oil, which was directly used in the next step.

### (2) Synthesis of compound 3

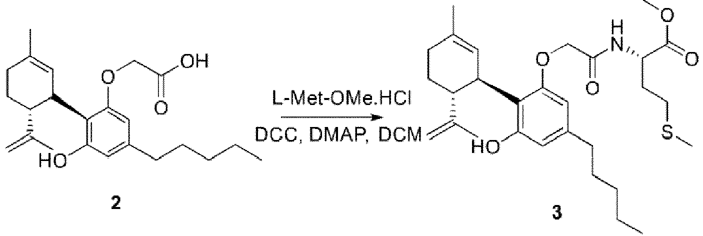

### Experimental procedures:

Compound **2** (9.5 g, 24.6 mmol, 1 eq), methionine methyl ester hydrochloride (6.4 g, 32 mol, 1.3 eq), and DMAP (4.5 g, 37 mol, 1.5 eq) were dissolved in dichloromethane (60 mL), DCC (7.6 g, 37 mmol, 1.5 eq) was added, and the mixture was stirred at room temperature overnight.

The reaction solution was filtered. The filter cake was washed with 50 mL of dichloromethane, and the filtrate was washed sequentially with 2 N hydrochloric acid and saturated sodium chloride, dried over anhydrous sodium sulfate, filtered, and concentrated. The crude product was subjected to column chromatography (n-hexane/dichloromethane: 5/1-1/1, n-hexane/dichloromethane/ethyl acetate: 1/1/0.1) to give compound **3** (6.6 g) as a yellow oil.

### (3) Synthesis of compound 4 (free acid)

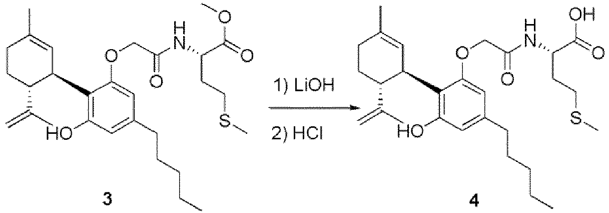

### Experimental procedures:

Compound **3** (6.6 g, 0.0128 mol, 1 eq) was dissolved in 80 mL of THF, and lithium hydroxide (1.6 g, 0.0383 mol, 3 eq) was dissolved in 10 mL of water. The above solutions were mixed, and the reaction solution was reacted at room temperature overnight.

The reaction solution was concentrated. 100 mL of ethyl acetate was added, and the mixture was adjusted to pH 2 with 2 N hydrochloric acid, and subjected to liquid separation. The organic phase was washed with saturated sodium chloride, dried over anhydrous sodium sulfate, filtered, and concentrated to give a crude product (5.5 g) as a yellow oil. The crude product was subjected to column chromatography (dichloromethane/methanol: 150/1-50/1) to give compound **4** (3.4 g) as a yellow oil.

### (4) Synthesis of compound E1 (sodium salt)

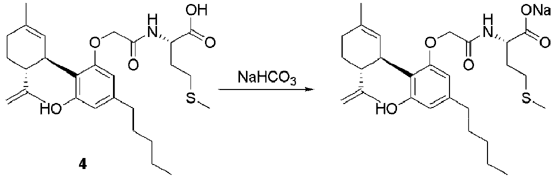

### Experimental procedures:

Compound **4** (3.4 g, 6.8 mmol, 1 eq) was dissolved in 20 mL of ethanol, and sodium bicarbonate (0.625 g, 7.4 mmol, 1.1 eq) was dissolved in 10 mL of water. The above solutions were mixed and stirred, heated to 50 °C, and reacted for 1 h.

The reaction solution was concentrated. 30 mL of ethanol was added, and the mixture was concentrated by rotary evaporation to dryness. The residue was washed with a mixed solvent of 60 mL of *n*-hexane and 20 mL of dichloromethane and dried to give E1 (2.4 g) as a pink solid (HPLC > 98%).

MS (ESI, positive): 504.1[M+H]+.

¹H-NMR (400MHz, CD₃OD): 6.30 (d, J=0.8Hz, 1H), 6.20 (s, 1H), 5.29 (s, 1H), 4.55-4.36 (m, 5H), 4.03-4.05 (m, 1H), 2.97-2.91 (m, 1H), 2.46 (t, J=7.6Hz, 2H), 2.42-2.36 (m, 2H), 2.26-2.16 (m, 2H), 2.05 (s, 3H), 2.04-1.91 (m, 2H), 1.80-1.74 (m, 2H), 1.63 (s, 3H), 1.561 (s, 3H), 1.61-1.53 (m, 2H), 1.37-1.32 (m, 4H), 0.90 (t, J=6.8Hz, 3H). The solubility of compound E1 in water was detected to reach 2.7 g/100 mL of water.

### Example 2: Synthesis of Compound E2

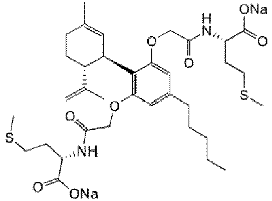

### 1. Synthetic route

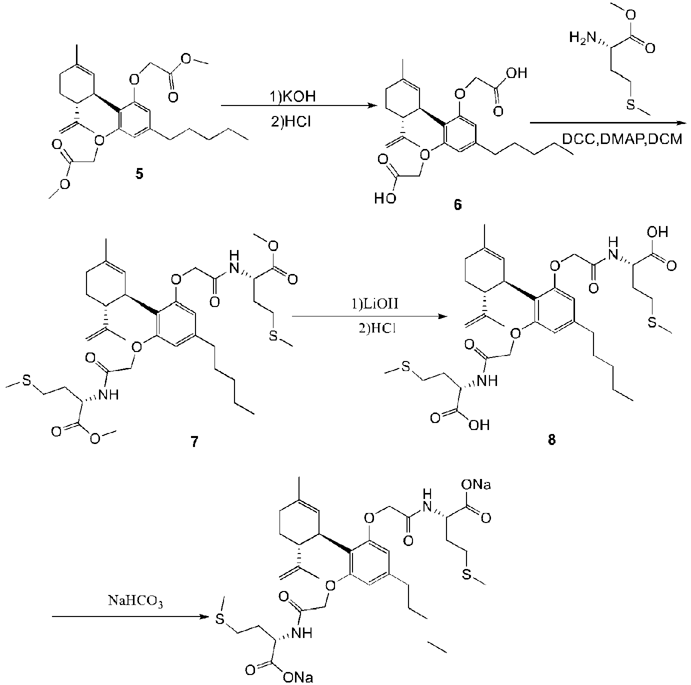

### 2. Synthetic method

### (1) Synthesis of compound 6

Compound 5 (10.5 g, 0.0229 mol, prepared in Example 1) was dissolved in 100 mL of methanol, potassium hydroxide (3.8 g, 0.0688 mol) was added, and the mixture was stirred and heated under reflux for 1 h. The reaction solution was concentrated. The residue was washed with 100 mL of ethyl acetate and filtered. The filter cake was added with 200 mL of ethyl acetate, and the resulting mixture was adjusted to pH 2 with 2 N diluted hydrochloric acid and subjected to liquid separation. The organic phase was washed with saturated sodium chloride, dried over anhydrous sodium sulfate, filtered, and concentrated to give compound **6** (9 g) as a yellow oil. MS(ESI): 431 [M+H]⁺.

### (2) Synthesis of compound 7

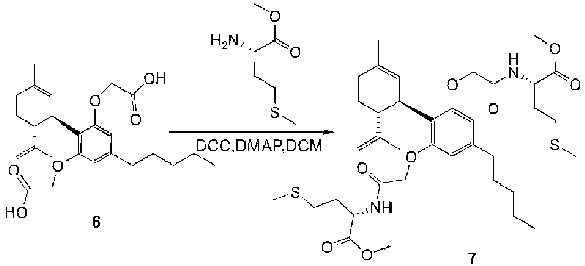

Compound **6** (6.3 g, 0.0145 mol), methionine methyl ester hydrochloride (11.7 g, 0.0586 mol), and DMAP (7.15 g, 0.0586 mol) were dissolved in 200 mL of dichloromethane, DCC (12 g, 0.0586 mmol) was added, and the mixture was reacted at room temperature overnight. The reaction solution was filtered. The filter cake was washed with 50 mL of dichloromethane, and the filtrate was washed sequentially with 2 N hydrochloric acid and saturated sodium chloride, dried over anhydrous sodium sulfate, concentrated, and subjected to column chromatography (*n-*hexane:dichloromethane = 5:1-1:1, n-hexane:dichloromethane:ethyl acetate = 1:1:0.1) to give compound **7** (6 g) as a yellow oil. MS (ESI): 721 [M+H]⁺.

### (3) Synthesis of compound 8

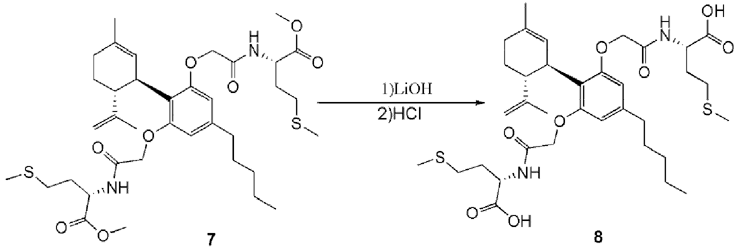

Compound 7 (10 g, 0.014 mol, 1 eq) was dissolved in 80 mL of THF, 8 mL of lithium hydroxide (1.7 g, 0.042 mol, 3 eq) solution was added, and the mixture was reacted at room temperature overnight. The reaction solution was concentrated, and 100 mL of ethanol was added for drying once. The residue was washed with *n*-hexane. After concentration, 100 mL of ethyl acetate was added, and the mixture was adjusted to pH 2 with 2 N hydrochloric acid, subjected to liquid separation, washed with saturated sodium chloride, dried over anhydrous sodium sulfate, and concentrated to give compound **8** (10 g) as a yellow solid. MS (ESI): 693 [M+H]⁺.

### (4) Synthesis of compound E2

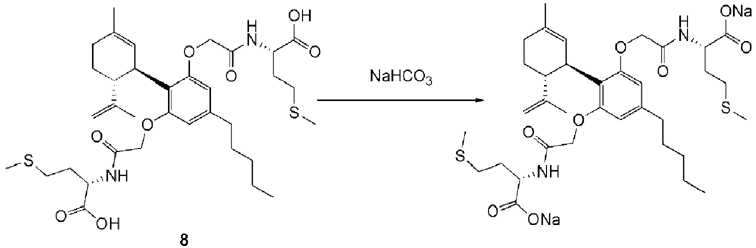

Compound **8** (10 g, 0.0144 mol) was dissolved in 80 mL of ethanol, and sodium bicarbonate (2.5 g, 0.0304 mol, 2.1 mol) was dissolved in 20 mL of water. The above solutions were mixed and stirred, and reacted at 50 °C for 1 h. The reaction solution was concentrated. 100 mL of ethanol was added, and the mixture was concentrated by rotary evaporation to dryness. The residue was washed with 20 mL of dichloromethane and dried to give E2 (8.1 g) as an off-white solid. MS (ESI) : 693 [M+H]⁺: ¹H-NMR (CD₃OD): 6.48 (s,2H), 5.32 (s,1H), 4.62-4.55 (m, 4H), 4.44-4.39 (m, 4H), 4.13-4.18 (m, 1H), 4.15 (d, 1H), 3.01-2.95 (m, 1H), 2.58-2.54 (t, 2H), 2.42-2.38 (t, 4H), 2.17-2.19 (m, 2H), 2.03 (s, 6H), 1.99-1.95 (m, 3H), 1.84-1.81 (t, 2H), 1.72 (s, 3H), 1.66 (s, 3H), 1.64-1.60 (m, 2H), 1.39-1.33 (m, 4H), 0.94-0.91 (t, 3H).

The solubility of compound E2 in water was detected to reach 20 g/100 mL of water.

### Example 3: Study on Antiepileptic Activity

### 1. Laboratory animals and some reagents

Wistar rats, SPF grade, weighing 240-260 g, aged 6-8 weeks, female, purchased from Shanghai Slac Laboratory Animal Co., Ltd. Feeding environment: the temperature was kept at 22 ± 1 °C, and the humidity was kept at 65%-70%, in a 12/12 hour light/dark illumination cycle, with free access to water.

PTZ, purchased from Sigma; sodium valproate, purchased from Sigma; and absolute ethanol, purchased from Sinopharm Chemical Reagent Co., Ltd.

### 2. Experimental grouping

Laboratory animals were randomly divided into 4 groups: model group, positive drug group, compound 1 group, and compound 2 group, with 8 animals in each group.

### 3. Experimental procedures

### (1) Modeling by intraperitoneal administration

After 1 week of acclimatization of all rats, modeling was started. A 1.75% PTZ solution was prepared with normal saline, and the prepared PTZ solution was injected intraperitoneally at a dose of 35 mg/kg. PTZ was injected once every other day, and behavioral changes of the rats were observed within 30 min after the PTZ injection, and the seizure grade was recorded.

During the experiment, after 11 consecutive injections of PTZ, the seizure grade was recorded. All rats had 5 consecutive recordings of grade 2 or above, and modeling for all rats was successful after 11 consecutive injections of PZT into all rats.

### (2) Treatment by intragastric administration

After the modeling was successful, the rats were randomly grouped. Treatment was started. All Wistar rats were weighed and doses for the intragastric administration were calculated. The model group was subjected to intragastric administration of an equal volume of normal saline; the positive drug group (valproate gavage, intragastric administration, 100 mg/kg), the compound 1 group (compound E1 prepared in Example 1, intragastric administration, 100 mg/kg), and the compound 2 group (compound E2 prepared in Example 2, intragastric administration, 100 mg/kg).

### (3) Ono's grading

Before the administration and on day 7 and day 14 after the administration, the prepared PTZ solution was injected intraperitoneally at a dose of 35 mg/kg 1 h after intragastric administration. Behavioral changes of the rats were observed within 30 min after the PTZ injection.

Behavioral seizure indexes before and after the administration were recorded and graded according to Ono's grading. The seizure latency, seizure grade and seizure duration were recorded.

**Table 1 Ono's grade**

| Ono's grade | Seizure manifestation |
|---|---|
| 0 | Without seizure |
| 1 | Nodding or head twitching |
| 2 | Systemic myoclonus |
| 3 | Head twitching with forelimbs clonus |
| 4 | Clonic seizure with hindlimbs standing |
| 5 | Falling down |
| 6 | Generalized tonic-clonic seizure |

At the end of the experiment, the brains and hippocampi of the laboratory animals were collected and frozen at - 80°C.

### 4. WB detection of expression of apoptosis-related proteins Bax and Bcl-2 in hippocampal tissues of rats:

### 4.1 Reagents, antibodies, and instruments

Some reagents, antibodies and instruments used in the experiment are shown in Tables 2-4, respectively.

**Table 2. Reagents**

| Reagent name | Source of reagent | Catalog No. |
|---|---|---|
| RIPA histiocyte rapid lysis buffer | Aladdin | R301899 |
| BCA protein quantification kit | thermo | 23225 |
| Acrylamide | Hushi | 79-06-1 |
| BIS-TRIS PROPANE | Vocko | 64431-96-5 |
| Hydrochloric acid | Hushi | 7647-01-0 |
| Sodium dodecyl sulfate | Hushi | 151-21-3 |
| 10% ammonium persulfate | Hushi | 7727-54-0 |
| Tetramethyl ethylenediamine | Hushi | 110-18-9 |
| SDS-PAGE protein loading buffer (5×) | Beyotime | P0015L |
| Pre-stained protein marker | thermo | 26616 |
| Ponceau S | Beyotime | P0022 |
| NC-based membrane | millipore | HATF00010 |
| Skimmed milk powder | Beyotime | P0216 |
| PBS (phosphate buffered saline) | Beyotime | ST476 |
| Tween20 | Hushi | 9005-64-5 |
| Luminescent liquid | Millipore | WBKLS0100 |

**Table 3. Antibodies**

| Antibody name | Source of antibody | Catalog No. |
|---|---|---|
| Bcl-2 | Proteintech | 26593-1-AP |
| Bax | Proteintech | 50599-2-Ig |
| GAPDH | Proteintech | 60004-1-Ig |
| Goat anti-rabbit HRP-labeled secondary antibody | Beyotime | A0208 |
| Goat anti-mouse HRP-labeled secondary antibody | Beyotime | A0216 |

**Table 4. Instruments**

| Instrument name | Source of instrument |
|---|---|
| Electrophoresis apparatus | BIO-RAD, model: mini protein 3 cell |
| Electrotransfer instrument | PS-9, Dalian Jingmai Biotechnology Co., Ltd. |
| Microplate reader | Finland, Labsystems microplate reader (model: MK3) |
| Pipettor | Gilson P-type pipettor (Pipetman) |
| Water bath kettle | Leica HI1210 water bath kettle |
| Imaging system | Tanon Tanon-5200 |

### 4.2 Sample preparation

The hippocampal tissue was cut into small pieces, the lysis buffer (protease and phosphatase inhibitors added to the lysis buffer) was added at a ratio of 150-250 µL:20 mg, and the mixture was homogenized with a homogenizer until complete lysis. The sample obtained after the lysis was centrifuged at 12,000 g at 4 °C for 15 min. The supernatant was taken, subjected to protein quantification, and stored in a refrigerator at -80 °C.

### 4.3 Protein quantification

(1) Standard curve plotting: a microplate was taken, and reagents were added according to the table below.

**Table 5. Reagents**

| Well number | 1 | 2 | 3 | 4 | 5 | 6 | 7 | 8 |
|---|---|---|---|---|---|---|---|---|
| Protein standard solution (µL) | 0 | 2 | 4 | 6 | 8 | 12 | 16 | 20 |
| Deionized water (µL) | 20 | 18 | 16 | 14 | 12 | 8 | 4 | 0 |
| Protein concentration (µg/µL) | 0 | 0.05 | 0.1 | 0.15 | 0.2 | 0.3 | 0.4 | 0.5 |

(2) According to the number of samples, a proper amount of BCA working solution was prepared by uniformly mixing BCA reagent A and reagent B according to a volume ratio of 50:1.
(3) 160 µL of BCA working solution was added to each well.
(4) The microplate was shaken on a shaker for 30 s, left to stand at 37 °C for 30 min, and subsequently measured for absorbance at 562 nm; a standard curve was plotted with the absorbance value as an abscissa and the protein concentration (µg/µL) as an ordinate.
(5) 2 µL of test protein and 18 µL of PBS (10-fold dilution) were added to the microplate, 160 µL of BCA working solution was added, and the microplate was shaken on a shaker for 30 s, left to stand at 37 °C for 30 min, and subsequently measured for the absorbance at 562 nm.
(6) According to the absorbance value of the test sample, the corresponding protein concentration (µg/µL) could be found on the standard curve, and the protein concentration was multiplied by a sample dilution factor (10) to obtain the actual concentration (µg/µL) of the sample.

### 4.4 Loading and electrophoresis

### (1) Sample loading

About 25 µg of the protein was loaded into each well, and the loading amount can be increased according to the experiment requirements. According to the results of protein quantification, the required protein was taken and a proper amount of sample loading buffer was added. After being heated in a boiling water bath for 10 min, the mixture was centrifuged to obtain a supernatant for loading. The prepared PAGE gel was added to an electrophoresis tank, and a proper amount of electrophoresis buffer was added. A comb was taken off, and loading wells were gently pipetted by using a pipettor to avoid residual gel remaining in the wells, which would affect sample loading. The prepared sample was added to the corresponding wells by using a pipettor, taking care not to allow the sample to overflow the loading well.

### (2) Electrophoresis

The general electrophoretic condition for the stacking gel is 80 V, 20 min, and for the separating gel is 120 V, 60 min. The voltage and the time can be adjusted according to specific experimental requirements. When the dye reached the bottom of the gel, the power supply was cut off to stop the electrophoresis, and the membrane transfer was performed in the next step.

### Membrane transfer

The membrane transfer is divided into wet transfer and semi-dry transfer. The semi-dry transfer was used in this experiment.

In the semi-dry membrane transfer, the sandwich arrangement was as follows: filter paper/glue/membrane/filter paper, and after being wetted with an electrotransfer buffer, the sandwich was placed directly between the positive and negative electrodes of the electrotransfer instrument, with the glue on the negative electrode and the membrane on the positive electrode. The electrotransfer buffer for the semi-dry membrane transfer is different from that for the wet membrane transfer, and an electrotransfer buffer containing 48 mM Tris, 39 mM glycine, 0.04% SDS and 20% methanol is recommended. The condition for the membrane transfer is 25 V, 30 min. Before the membrane transfer, the PVDF membrane was soaked in methanol for 1-2 min (the NC-based membrane was soaked in an electrotransfer buffer for 10-20 min), and subsequently incubated in an ice-cold electrotransfer buffer for 5 min. The gel is required to be equilibrated in an ice-cold electrotransfer buffer for 3-5 min, or it will lead to deformation of bands when transferring.

### Detection of proteins on membrane

For determining whether the membrane transfer is successful, the membrane can be stained with ponceau. A ponceau staining working solution was obtained by diluting 2% ponceau stock solution in a 1:10 ratio, i.e., adding 9 volumes ofddH₂O.

Staining method: The membrane was washed once in TBST, and subsequently placed in the ponceau staining working solution and shaken for staining for 5 min at room temperature. The membrane was washed with a large amount of water until the water became clarified and colorless and the protein bands were clear (the membrane could be re-washed with TBST or water and subsequently stained), and the PVDF membrane was required to be reactivated by methanol, washed with TBST, and subsequently blocked.

### 4.5 Membrane blocking and antibody incubation

(1) Blocking: the membrane was blocked with 5% skimmed milk powder (BSA for detection of phosphorylated protein) at room temperature for 1 h or at 4 °C overnight.
(2) Primary antibody: The antibodies were diluted according to Bcl-2 1:500 Bax 1:5000 GAPDH 1:30,000 of the instructions. The diluted antibodies were each added to the blocking solution and diluted to the desired concentration, and incubated with the membrane at room temperature for 2 h or at 4 °C overnight.
(3) Secondary antibody: The membrane incubated with the primary antibody was washed 3 times with TBST for 5 min each. The HRP-labeled secondary antibody was subsequently diluted in a 1:1000 ratio according to the amount and incubated with the membrane at 37 °C for 1 h. The membrane was washed 3 times with TBST for 5 min each.

### 4.6 Color development

ECL chemiluminescence detection: An ECL luminescent liquid was prepared. ECL luminescent liquid A and ECL luminescent liquid B were uniformly mixed in equal volume according to the amount, added to the front of the membrane, and incubated in the dark for 5 min in a dark room. The color developing liquid was poured off, the remaining color developing liquid was carefully absorbed with paper, and the membrane was covered with a layer of smooth transparent paper. The membrane was placed into an imaging system for scanning.

### 5. Determination of contents of γ-aminobutyric acid, glutamic acid, glycine, and aspartic acid in rat brain tissue by the kit method

### 5.1 Experimental materials

The kits and instruments used in the experiment are shown in the table below.

**Table 6. Kit information**

| Antibody name | Source of antibody | Catalog No. |
|---|---|---|
| Colorimetric L-Aspartate(Aspartic Acid) Assay Kit | AAT Bioquest | 13828 |
| Glycine Assay Kit | Sigma | MAK261 |
| Glutamic acid (Glu) content detection kit | NanJing JianCheng | BC1585 |
| Rat γ-Aminobuytyric (GABA) Elisa Kit | NanJing JianCheng | H168 |
| Protein quantification (BCA method) test kit | NanJing JianCheng | A045-3 |

**Table 7. Main instruments and consumables**

| Instrument name | Manufacturer | Model |
|---|---|---|
| Microplate reader | Finland (Labsystems Multiskan MS) corporation | Model 352 |
| Microplate washer | Finland (Thermo Labsystems) corporation | AC8 |
| Centrifuge | High speed microcentrifuge (domestic) | TG16W |
| Thermostatic incubator | Insulated thermostatic incubator (domestic) | Model GNP-9080 |
| Pipettor | P-type pipettor (Gilson Company, Inc.) | P2, P10, P20, P100, P200, |

### 5.2 Experimental procedures

The experimental procedures and methods were referred to the kit instructions.

### 6. Experimental results

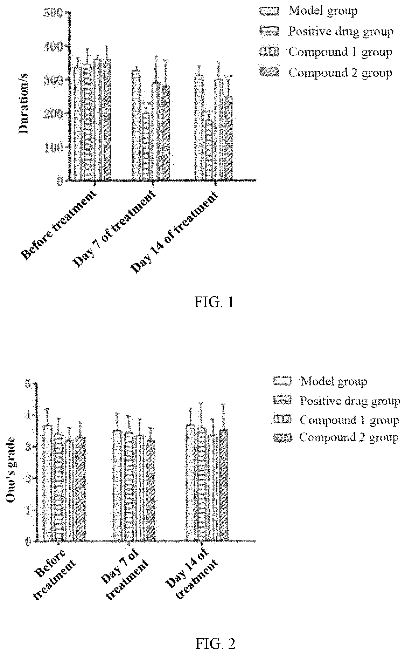

(1) The duration of epileptic seizure in PTZ-kindling epileptic rats before and after treatment is shown in the table below and in FIG. 1.

**Table 8. Duration of epileptic seizure in PTZ-kindling epileptic rats before and after treatment**

| Group | Duration before treatment/s | Duration on day 7 of treatment/s | Duration on day 14 of treatment/s |
|---|---|---|---|
| Model group | 338.33±27.16 | 327.50±12.01 | 311.83±29.02 |
| Positive drug group | 347.25±44.10 | 200.29±18.43 | 178.71±16.96 |
| Compound 1 group | 359.83±14.41 | 290.50±66.93 | 299.67±39.25 |
| Compound 2 group | 358.57±40.33 | 280.17±65.01 | 250.33±48.64 |

The results showed that: in terms of duration of PTZ-kindling epileptic seizure before and after treatment, through the comparison of the data, it was found that there were no significant differences in the duration among the groups before treatment, with the duration of seizure being about 350 s; there were significant differences among the groups as they were introduced in the treatment stage, with the positive drug group showing a decrease in the duration of seizure after 7 days and 14 days of treatment, which was statistically significant. The compound groups also showed a significant decrease in the duration of seizure after 7 days of treatment as compared to that before treatment, which was statistically significant. Compound 2 showed a significant decrease in the duration of seizure on day 14 of subsequent treatment as compared to that on day 7 of treatment, which was statistically significant; compound 1 showed no change in the duration of seizure on day 14 as compared to that on day 7 of treatment.

(2) Ono's grade for behavioral seizure indexes of PTZ-kindling epileptic rats before and after treatment is shown in the table below and in FIG. 2.

**Table 9. Ono's grade for behavioral seizure indexes of PTZ-kindling epileptic rats before and after treatment**

| Group | Ono's grade before treatment | Ono's grade on day 7 of treatment | Ono's grade on day 14 of treatment |
|---|---|---|---|
| Model group | 3.67±0.52 | 3.50±0.55 | 3.67±0.52 |
| Positive drug group | 3.38±0.52 | 3.43±0.53 | 3.57±0.79 |
| Compound 1 group | 3.17±0.41 | 3.33±0.52 | 3.33±0.52 |
| Compound 2 group | 3.29±0.49 | 3.17±0.41 | 3.50±0.84 |

The results showed that: behavioral seizure indexes before and after treatment were recorded according to Ono's grade; PTZ was injected for the epileptic kindling model after modeling and during the treatment, and Ono's grade of seizure was between grades 3-4 for all groups, with no significant differences among the groups. After 14 days of treatment, there were also no significant differences among the groups.

During the experiment, the highest grade for epileptic seizure in rats was recorded according to the Ono's grading. The epileptic seizure was a progressive process during the experiment, and the highest grade of epileptic seizure would have a duration during the epileptic seizure. The duration of each seizure grade was recommended to be recorded subsequently.

During the experiment, it was found that when the seizure was at grade 5-6 according to Ono's grading, most of the rats would die without taking measures, and those rats that did not show death would die next in the case of PTZ kindling.

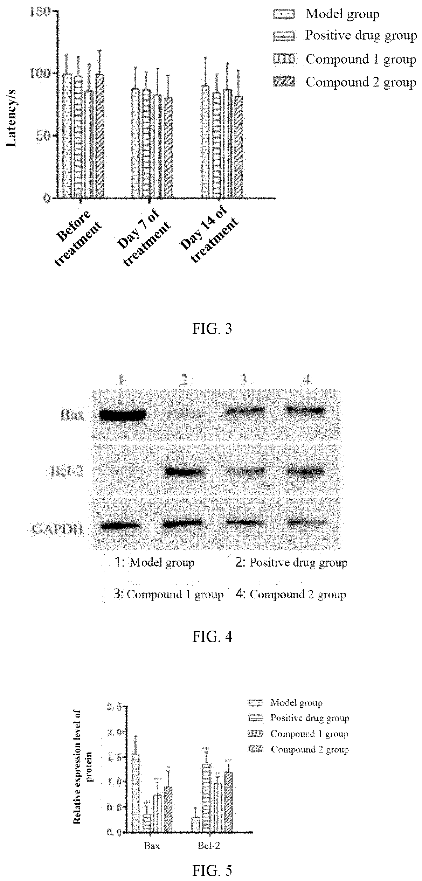

(3) The latency to epileptic seizure in PTZ-kindling epileptic rats before and after treatment is shown in the table below and in FIG. 3.

**Table 10. Latency to epileptic seizure in PTZ-kindling epileptic rats before and after treatment**

| Group | Latency before treatment/s | Latency on day 7 of treatment/s | Latency on day 14 of treatment/s |
|---|---|---|---|
| Model group | 99.14±15.52 | 87.67±16.82 | 90.00±22.73 |
| Positive drug group | 97.38±15.86 | 87.00±13.90 | 84.43±14.77 |
| Compound 1 group | 85.75±21.45 | 82.83±20.90 | 87.00±20.96 |
| Compound 2 group | 99.00±1914 | 80.50±17.51 | 81.17±21.31 |

The results showed that: in terms of latency to PTZ-kindling epileptic seizure before and after treatment, the mean value of latency to PTZ-kindling epileptic seizure was calculated to be 80-100 s before and during treatment, and the data for seizure latency indicated that the time for PTZ-kindling epileptic seizure was 60-120 s. There was no significant correlation between the groups, and no significant trend of change in the latency of seizure over time was observed between the groups.

(4) The results for WB detection of changes in apoptosis-related proteins Bax and Bcl-2 in hippocampal tissues of rats are shown in FIGs. 4 and 5.

The experimental results showed that there was high expression of Bax protein related to apoptosis promotion in the hippocampal tissues of rats in the model group, while there was low expression of Bax protein related to apoptosis promotion in the hippocampal tissues of rats in the positive drug group and the model group, which were statistically significant. Compound E1 and compound E2 both showed significant down-regulation of Bax protein expression, which was statistically significant.

In contrast, in the detection of the anti-apoptotic Bcl-2 protein in the hippocampal tissues of rats in the groups, the experimental results showed that the expression of Bcl-2 protein in the model group was significantly lower than that in the other groups, the expression of Bcl-2 protein in the positive drug group was significantly higher than that in the other groups, and the expression of Bcl-2 protein in compound E1 and compound E2 of the compound groups was significantly higher than that in the model group, which were statistically significant.

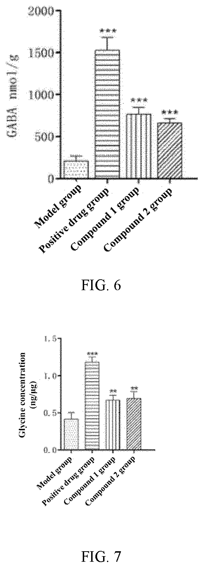

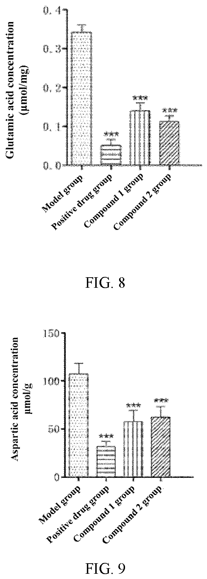

(5) The results for the determination of contents of γ-aminobutyric acid, glutamic acid, glycine, and aspartic acid in the homogenate of brain tissues of rats are shown in the table below and FIGs. 6-9.

**Table 11. Results for the determination of contents of γ-aminobutyric acid, glutamic acid, glycine, and aspartic acid in the homogenate of brain tissues of rats**

| Group | γ-aminobutyric acid (nmol/g) | Glutamic acid (µmol/mg) | Glycine (ng/µg) | Aspartic acid (µmol/g) |
|---|---|---|---|---|
| Model group | 214.18±60.03 | 0.34±0.02 | 0.42±0.09 | 107.28±11.12 |
| Positive drug group | 1528.29±152.58 | 0.05±0.02 | 1.18±0.06 | 31.66±5.57 |
| Compound 1 group | 771.17±81.98 | 0.14±0.02 | 0.67±0.07 | 58.10±11.71 |
| Compound 2 group | 663.91±51.53 | 0.11±0.01 | 0.69±0.09 | 62.53±10.95 |

The contents of excitatory amino acids (glutamic acid and aspartic acid) and inhibitory neurotransmitters (γ-aminobutyric acid and glycine) in the homogenate of brain tissues were determined by an ELISA kit.

The ELISA experiment results showed that the contents of γ-aminobutyric acid and glycine in the homogenate of brain tissues of the model group were low, while the contents of glutamic acid and aspartic acid were high.

The positive drug group and the compound group both showed a decrease in the expression levels of excitatory amino acids as compared to the model group, which was statistically significant; the positive drug group and the compound group showed an increase in the expression levels of inhibitory neurotransmitters γ-aminobutyric acid and glycine as compared to the model group, which was statistically significant.

### 7. Summary

During the treatment of the PTZ-kindling epileptic model of rats, the therapeutic effects of the positive drug and the compounds on rats were significantly different in the duration of PTZ-kindling epileptic seizure in rats, and had a trend towards action in the latency and the extent of epilepsy modeling, but no significant differences.

Compared with the model group, the 2 compound groups both showed a significant reduction in the duration of epileptic seizure on day 7 and day 14, which was statistically significant. On day 7 of treatment, the groups were ranked in terms of the duration of epileptic seizure as follows: compound E2 > compound E1, and on day 14 of treatment, the groups were ranked in terms of the duration of epileptic seizure as follows: compound E2 > compound E1.

The apoptosis-related proteins Bax and Bcl-2 in hippocampal tissues of rats on day 14 of treatment, and the contents of excitatory amino acids (glutamic acid and aspartic acid) and inhibitory neurotransmitters (γ-aminobutyric acid and glycine) in the homogenate of brain tissues were compared.

### Example 4: Study on Treatment for Parkinson's Disease

### 1. Laboratory animals and some reagents

C57BL6 mice, SPF grade, weighing 18-20 g, aged 6-8 weeks, male, purchased from Shanghai Slac Laboratory Animal Co., Ltd. Feeding environment: the temperature was kept at 22 ± 1 °C, and the humidity was kept at 65%-70%, in a 12/12 hour light/dark illumination cycle, with free access to water.

MPTP, purchased from Sigma; amantadine, purchased from Sigma; and absolute ethanol, purchased from Sinopharm Chemical Reagent Co., Ltd.

### 2. Experimental grouping

Laboratory animals were randomly divided into 5 groups: blank group, model group, positive drug group, compound 1 group, and compound 2 group, with 8 animals in each group.

### 3. Experimental procedures

(1) SPF-grade C57 mice aged 6-8 weeks were purchased and acclimatized for one week, and subsequently the modeling was started.
(2) MPTP (25 mg/kg) was intraperitoneally injected once a day for 7 consecutive days. The blank group was left untreated.
(3) After modeling, administration was started. The positive drug group was subjected to intragastric administration of amantadine (40 mg/kg), and the test compound groups were subjected to intragastric administration of the drug at a determined dose (100 mg/kg). The intragastric administration was performed once a day for 14 consecutive days.
(4) Behavioral test was first performed by balance beam test scoring.

A beam of 80 cm long and 2.5 cm wide was horizontally fixed at a height of 10 cm from the table top, and subsequently, the animals were allowed to walk on the beam.

The scoring scale was as follows: being able to jump on the balance beam and to walk freely without falling down: 0 points; being able to jump on the balance beam, but having a probability of tumbling down of less than 50% when walking on the balance beam: 1 point; being able to jump on the balance beam, but having a probability of tumbling down of greater than 50% when walking on the balance beam: 2 points; being able to jump on the balancing beam with the help of the normal side of the body, but failing to get help from the hind limb on the paralyzed side to move forward: 3 points; being unable to walk on the balance beam, but being able to sit on the beam: 4 points; falling off quickly when being placed on the beam: 5 points. (5) After the scoring, the sampling was performed.

After the mice were sacrificed quickly, the brains of the mice were taken and the cerebral palliums (CPs) of the mice were isolated. After exposing the brain striatum of the mice, the striatum was weighed and homogenized after adding PBS according to a proportion. The supernatant was isolated and detected for the content of dopamine in the brain striatum.

### 4. Determination of content of dopamine in brain striatum by the kit method

### 4.1 The kits and some instruments used in the experiment are shown in the table below.

**Table 12. Kit information**

| Antibody name | | Source of antibody | | Catalog No. |
|---|---|---|---|---|
| Mouse dopamine (DA) detection kit | | NanJing JianCheng | | H170 |
| Protein quantification (BCA method) test kit | | NanJing JianCheng | | A045-3 |

**Table 13. Main instruments and consumables**

| Instrument name | Manufacturer | | Model |
|---|---|---|---|
| Microplate reader | Finland (Labsystems Multiskan MS) corporation | | Model 352 |
| Microplate washer | Finland (Thermo Labsystems) corporation | | AC8 |
| Centrifuge | High speed microcentrifuge (domestic) | | TG16W |
| Thermostatic incubator | Insulated thermostatic incubator (domestic) | | Model GNP-9080 |
| Pipettor | P-type pipettor (Gilson Company, Inc.) | | P2, P10, P20, P100, P200, |

### 4.2 Protein quantification

(1) Standard curve plotting: a microplate was taken, and reagents were added according to the table below.

**Table 14. Reagents**

| Well number | 1 | 2 | 3 | 4 | 5 | 6 | 7 | 8 |
|---|---|---|---|---|---|---|---|---|
| Protein standard solution (µL) | 0 | 2 | 4 | 6 | 8 | 12 | 16 | 20 |
| Deionized water (µL) | 20 | 18 | 16 | 14 | 12 | 8 | 4 | 0 |
| Protein concentration (µg/µL) | 0 | 0.05 | 0.1 | 0.15 | 0.2 | 0.3 | 0.4 | 0.5 |

(2) According to the number of samples, a proper amount of BCA working solution was prepared by uniformly mixing BCA reagent A and reagent B according to a volume ratio of 50:1.
(3) 160 µL of BCA working solution was added to each well.
(4) The microplate was shaken on a shaker for 30 s, left to stand at 37 °C for 30 min, and subsequently measured for absorbance at 562 nm. A standard curve was plotted with the absorbance value as an abscissa and the protein concentration (µg/µL) as an ordinate.
(5) 2 µL of test protein and 18 µL of PBS (10-fold dilution) were added to the microplate, 160 µL of BCA working solution was added, and the microplate was shaken on a shaker for 30 s, left to stand at 37 °C for 30 min, and subsequently measured for the absorbance at 562 nm.
(6) According to the absorbance value of the test sample, the corresponding protein concentration (µg/µL) could be found on the standard curve, and the protein concentration was multiplied by a sample dilution factor (10) to obtain the actual concentration (µg/µL) of the sample.
(7) The experimental procedures and methods for the determination of dopamine in mice were referred to the kit instructions.

### 5. Determination of tyrosine hydroxylase (TH) cells in substantia nigra (SubN) by immunohistochemistry

### 5.1 Experimental materials

**Table 15. Main reagents**

| Reagent name | Manufacturer | Catalog No. |
|---|---|---|
| Paraffin | Sinopharm Chemical Reagent Co., Ltd. | 69018961 |
| Formaldehyde | Sinopharm Chemical Reagent Co., Ltd. | 10010018 |
| Xylene | Sinopharm Chemical Reagent Co., Ltd. | 10023418 |
| Absolute ethanol | Sinopharm Chemical Reagent Co., Ltd. | 10092680 |
| 30%H₂O₂ | Sinopharm Chemical Reagent Co., Ltd. | 10011218 |
| Broad-spectrum secondary antibody | Shanghai Long Island Biotec. Co., Ltd. | D-3004 |
| DAB concentrated kit | Shanghai Long Island Biotec. Co., Ltd. | FL-6001 |
| Hematoxylin | BASO | 714094 |
| Neutral resin | Beijing Solarbio | G8590 |
| Anti-TyrosineHydroxylase | abeam | ab137869 |

**Table 16. Main instruments and consumables**

| Instrument name | Manufacturer | Model |
|---|---|---|
| Upright microscope | NIKON | ECLIPSE Ni |
| Pipettor | P-type pipettor (Gilson Company, Inc.) | P2, P10, P20, P100, P200, P1000 |
| Constant temperature oven | Shanghai Yiheng Technology Instrument Co., Ltd. | DHG-9023A |
| Paraffin slicing machine | Leica | SQ2125 |
| Slice spreading machine | Leica | PPTHK-21B |
| Micrograph analysis system | NIKON | DS-Ri2 |

### Preparation of solutions

(1) PBS solution
   8 g of NaCl, 0.2 g of KCl, 1.44 g of Na₂HPO₄, and 0.24 g of KH₂PO₄ were dissolved in 600 mL of ddH₂O. The solution was adjusted to pH 7.4 with HCl, and after the volume was made up to 1 L, the solution was filtered through a filter, autoclaved, and stored at room temperature.
(2) 0.1 mol/L sodium citrate buffer
   3.8 mL of 0.1 mol/L citric acid and 16.2 mL of 0.1 mol/L sodium citrate.
   Citric acid C₆H₈O₇·H₂O: molecular weight: 210.14, 21.01 g/L for a 0.1 mol/L solution.
   Sodium citrate Na₃C₆H₅O₇·2H₂O: molecular weight: 294.12, 29.41 g/mL for a 0.1 mol/L solution.

### 5.2 Experimental procedures

### 5.2.1 Sample embedding and fixing

(1) Sampling and fixing
   The tissue should be cut using sharp knives and scissors. When cutting a tissue mass, the tissue was cut by pulling backwards from the root of the knife. The tissue mass had a thickness of about 0.2-0.3 cm and a size of 1.5 cm × 1.5 cm × 0.3 cm. The prepared tissue mass was fixed in 10% formalin for 48 h.
(2) Washing and dehydration
   The fixed tissue was washed with running water to remove residual fixing liquid and impurities. The tissue mass was dehydrated in stages with different concentrations of ethanol (50%, 70%, 85%, 95%, up to pure alcohol (absolute ethanol)), each stage for 2 h. Dehydration must be performed in a capped flask to prevent high concentrations of ethanol from absorbing moisture in the air, resulting in reduced concentrations and incomplete dehydration. The material to be preserved can remain in 70% ethanol when being dehydrated with it. For long-term preservation, an equivalent amount of glycerol can be added.
(3) Transparentizing
   The tissue mass was placed into a mixed solution of pure ethanol and xylene in an equivalent volume and left to stand for 2 h, subsequently placed into pure xylene and left to stand for two hours, and placed into pure xylene and left to stand for another two hours. After transparentizing, the effect of the previous dehydration of the material can be shown. If the dehydration is complete, the tissue is in a transparent state, and if there is a white cloud-shaped substance in the tissue, it means that the dehydration is incomplete and must be reworked, but the effect of rework is often not good. When xylene is used for transparentizing, it should be prevented from volatilizing and absorbing moisture in the air, and it should be kept in an anhydrous state.
(4) Paraffin immersion
   The paraffin immersion must be performed in a thermostat. The tissue mass material was first immersed in a mixed solution of molten paraffin and xylene in equal volume for 1-2 h and subsequently immersed in 2 parts of molten paraffin solutions for 3 h each.
(5) Embedding
   Embedding is the encapsulation of the paraffin-impregnated tissue mass with paraffin. Specifically, a paper box was first prepared, molten paraffin was added to the box, a tissue mass was quickly clamped by using a pair of preheated tweezers, and placed flat at the bottom of the paper box with a cut surface facing downwards. Subsequently, the paper box was slightly lifted up and placed flat in cold water, immediately pressed into the water after the paraffin on the surface was solidified for quickly cooling and solidifying the paraffin, and taken out after 30 min.
(6) Slicing
   The paraffin block was placed in a refrigerator at -20 °C for at least 30 min before slicing to increase hardness. The slicing knife was arranged on a knife rest of the slicing machine and was tightly fixed, a paraffin block base or a paraffin block was fixed, and the paraffin block and the knife were adjusted to proper positions, with the knife edge at an angle of 5 degrees to the surface of the paraffin block. The thickness for slicing on the slicing machine was adjusted to 4-7 µm before the slicing was performed.

### 5.2.2 Immunohistochemical staining

(1) Slice baking and deparaffinating
   A slide was baked in a constant temperature oven at 65 °C for slice baking for 30 min, soaked in xylene I for 15 min, and subsequently in xylene II for 15 min.
(2) Hydration
   The deparaffinated slice was sequentially soaked in 100% alcohol, 95% alcohol, 85% alcohol, and 75% alcohol for 5 min, and washed with tap water for 10 min.
(3) Antigen retrieval
   The slice was retrieved at high pressure in 0.01 M sodium citrate buffer for 15 min, and subsequently washed with 0.02 M PBS 3 times for 3 min each after natural cooling.
(4) Blocking
   The slide was placed in 3% H₂O₂ and incubated in a wet box for 10 min to eliminate the activity of endogenous peroxidase. The slide was washed with 0.02 M PBS 3 times for 3 min each.
(5) Incubation with primary antibody
   A primary antibody (1:2000 dilution) was added dropwise, and the slide was incubated in a wet box, and left to stand at room temperature for 1 h (or incubated at 4 °C overnight). The slide was washed with 0.02 M PBS 3 times for 3 min each.
(6) Incubation with secondary antibody
   HRP was added to label the broad-spectrum secondary antibody, and the slide was incubated in a wet box, and left to stand at room temperature for 20-30 min. The slide was washed with 0.02 M PBS 3 times for 3 min each.
(7) Color development
   The slice was stained with DAB, and when a color change was observed in the slice, the staining liquid was immediately washed away with tap water.
(8) Hematoxylin staining
   The slice was re-stained with hematoxylin for 3 min, differentiated with 1% hydrochloric acid alcohol, and observed under a microscope for controlling the staining degree. The slice was washed with tap water for 10 min and placed in an oven at 65 ° C for removing water by drying.
(9) Transparentizing and sealing
   The slide was placed in xylene for transparentizing 2 times for 3 min each, sealed with a neutral resin, and placed in an oven at 65 ° C for 15 min.

### 5.2.3 Image acquisition and analysis

The slice was photographed through the microscope, and the relevant parts of the sample were collected and analyzed to calculate the positive area.

### 6. Experimental results

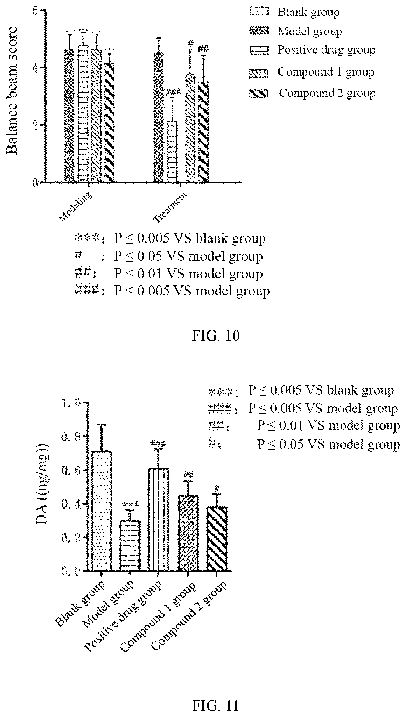

(1) The results for the behavioral balance beam score of the model of Parkinson's disease are shown in the table below and in FIG. 10.

**Table 17. Results for behavioral balance beam score of the model of Parkinson's disease**

| Group | After modeling | After treatment |
|---|---|---|
| Blank group | 0 | 0 |
| Model group | 4.63±0.52*** | 4.50±0.53 |
| Positive drug group | 4.75±0.46*** | 2.13±0.83### |
| Compound 1 group | 4.63±0.52*** | 3.75±0.89 # |
| Compound 2 group | 4.13±0.35*** | 3.50±0.93# # |

The mice were intraperitoneally injected with MPTP for the preparation of the model of Parkinson's disease, and subjected to behavioral evaluation by balance beam scoring. The balance beam scoring was performed 7 days after intraperitoneal injection of MPTP. Except for the blank group, which was not modeled and had a balance beam score of 0 points, all other groups of mice had scores of 5-6 points, with P ≤ 0.05 for the comparison of the groups with the blank group, which was statistically significant, proving that the modeling was successful.

After subsequent treatment with the positive drug and the compounds, the model group had a balance beam score of 4-5 points and the positive drug group had a balance beam score of 2-3 points. The compound treatment groups had balance beam scores of 3-4 points. The positive drug group and the compound groups showed a significant descending trend as compared to the model group, with P ≤ 0.05, which was statistically significant.

After treatment, the groups were ranked in terms of the scores as follows: blank group < positive drug group < compound 2 group < compound 1 group < model group. (2) The content of dopamine in striatum of the model of Parkinson's disease after treatment is shown in the table below and in FIG. 11.

**Table 18. Content of dopamine in striatum of the model of Parkinson's disease after treatment**

| Group | DA (ng/mg) |
|---|---|
| Blank group | 0.71±0.16 |
| Model group | 0.30±0.07*** |
| Positive drug group | 0.61±0.12## # |
| Compound 1 group | 0.45±0.09# # |
| Compound 2 group | 0.38±0.08 # |

Dopamine is used as neurotransmitter to regulate various physiological functions of the central nervous system. The effect of drug treatment of the model of Parkinson's disease was determined by determining the content of dopamine in striatum of the model of Parkinson's disease of treated mice.

The degree of decrease in the content of dopamine in the model was determined by comparing the content of dopamine in the groups with that in the blank group. Except for the positive drug group, all other groups showed a significant decrease in the content of dopamine, with P ≤ 0.05, which was statistically significant.

Subsequently, through the comparison of the content of dopamine in the compound groups with that in the model group, the groups showed an increase in the content of dopamine as compared to the model group, with P ≤ 0.01, which was statistically significant, indicating that the compound groups had the function of increasing the content of dopamine in the brain.

The groups were ranked in terms of the content of dopamine in striatum as follows: blank group > positive drug group > compound 1 group > compound 2 group > model group. (3) The experimental results for the determination of tyrosine hydroxylase (TH) cells in substantia nigra (SubN) by immunohistochemistry are shown in the table below and in FIG.s 12 and 13.

**Table 19. Experimental results for determination of tyrosine hydroxylase (TH) cells in substantia nigra (SubN) by immunohistochemistry**

| Group | TH cell positive rate (%) |
|---|---|
| Blank group | 34.27±5.08 |
| Model group | 12.74±1.71*** |
| Positive drug group | 20.82±5.77## |
| Compound 1 group | 16.92±3.13## |
| Compound 2 group | 15.18±3.37 |

Tyrosine hydroxylase (TH) is a monooxygenase, which is a rate-limiting enzyme in the first step of a series of reactions in which organisms are catalyzed to synthesize L-dopamine (DA) themselves, and is expressed only in the cytoplasm. TH is abundant in neurons. A neuron which is positive for the TH immune response of the midbrain is a DA neuron due to the lack of dopamine-β-hydroxylase in the midbrain, which can be used as a marker of dopaminergic neurons in the brain. In the body, L-tyrosine is used to generate levodopa under the catalysis of tyrosine hydroxylase, and levodopa is subjected to carboxyl removal under the catalysis of aromatic decarboxylase to finally generate L-dopamine. Due to the important role of tyrosine hydroxylase in dopamine synthesis, its function loss or underexpression directly affects the synthesis and secretion of dopamine. Dopamine is an important neurotransmitter, and the inability to synthesize or insufficient secretion of dopamine by dopaminergic neurons leads to Parkinson's disease.

The experimental results showed that in the mouse model of Parkinson's disease established by the intraperitoneal injection of MPTP, the positive rate of tyrosine hydroxylase (TH) cells in substantia nigra (SubN) of the mice was decreased, with P ≤ 0.05, which was statistically significant. The positive drug group showed a significant increase in the positive rate of tyrosine hydroxylase (TH) cells in substantia nigra (SubN) as compared to the model group, indicating that the modeling was successful.

Through the comparison of the treatment effect on the mouse model of Parkinson's disease of the compound groups with that of the model group, the compound groups showed an increase in the positive rate of tyrosine hydroxylase (TH) cells in substantia nigra (SubN) as compared to the model group, with P ≤ 0.01 for the comparison of the positive rate of tyrosine hydroxylase (TH) cells in substantia nigra (SubN) of the compound 1 group with that of the model group, which was statistically significant.

Through the comparison of the positive rates of tyrosine hydroxylase (TH) cells in substantia nigra (SubN) of the groups after treatment, the groups were ranked as follows: blank group > positive drug group > compound 1 group > compound 2 group > model group.

### 7. Summary

In the experiment, the mouse model of Parkinson's disease was established by the intraperitoneal injection of MPTP (25 mg/kg) once a day for 7 consecutive days, and was screened by the balance beam scoring.

Subsequently, the mice were subjected to treatment by intragastric administration of the positive drug and the compounds. After the treatment by intragastric administration for 14 consecutive days, the differences between the compound groups and the blank and model groups were identified by analyzing the balance beam scores of the groups after treatment, determining the content of dopamine in striatum by ELISA, determining the positive rate of tyrosine hydroxylase (TH) cells in substantia nigra (SubN) by immunohistochemistry, and other assay methods. Subsequently, statistical analysis was performed to analyze the effectiveness of the compounds.

Through the evaluation of the balance beam score after treatment, the positive drug group and the compound groups showed a significant descending trend as compared to the model group, with P ≤ 0.05, which was statistically significant.

Subsequently, through the comparison of the content of dopamine in the compound groups with that in the model group, the groups showed an increase in the content of dopamine as compared to the model group, with P ≤ 0.01, which was statistically significant.

Through the comparison of the positive rates of the positive drug group and the compound groups with that of the model group after the treatment of the mouse model of Parkinson's disease with the compounds and the positive drug, it was shown that P ≤ 0.01 for the comparison of the positive rate of tyrosine hydroxylase (TH) cells in substantia nigra (SubN) of the compound 1 group with that of the model group, which was statistically significant. Through overall analysis of the comparison of the compound groups with the model group, the compound groups both had a certain therapeutic effect, and the compound 1 group had a better therapeutic effect through the comparison of detection indexes.

The above description is only for the purpose of illustrating the preferred examples of the present invention, and is not intended to limit the scope of the present invention. Any modifications, equivalents, and the like made without departing from the spirit and principle of the present invention shall fall in the protection scope of the present invention.

The foregoing examples and methods described in the present invention may vary based on the abilities, experience, and preferences of those skilled in the art.

The certain order in which the steps of the method are listed in the present invention does not constitute any limitation on the order of the steps of the method.

## Claims

1. A compound, or a pharmaceutically acceptable salt, a stereoisomer, an ester, a prodrug or a solvate thereof, wherein the compound has the following structure: 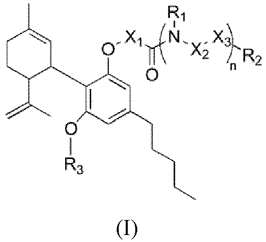 wherein,
X₁ and X₂ are independently selected from: a single bond, substituted or unsubstituted alkylene, alkylene spaced by one or more -C(=O)O-, alkylene spaced by one or more -OC(=O)-, and alkylene spaced by one or more -C(=O)NH-;
X₃ is selected from: 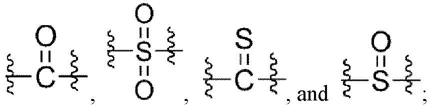
R₁ is selected from: H, alkyl, and cycloalkyl;
R₂ is selected from: OH and alkoxy;
n is an integer of 1-5;
R₃ is selected from: Hand 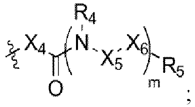
wherein, X₄ and X₅ are independently selected from: a single bond, substituted or unsubstituted alkylene, alkylene spaced by one or more -C(=O)O-, alkylene spaced by one or more -OC(=O)-, and alkylene spaced by one or more -C(=O)NH-;
X₆ is selected from: 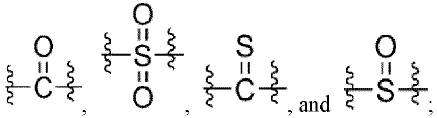
R₄ is selected from: H, alkyl, and cycloalkyl;
R₅ is selected from: OH and alkoxy;
m is an integer of 1-5.

2. The compound according to claim 1, wherein X₁ and X₄ are independently selected from: a single bond or linear and branched C1-6 alkylene;
preferably, X₁ is methylene;
preferably, X₄ is methylene.

3. The compound according to claim 1, wherein X₂ has the following structure: 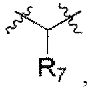 wherein R₇ is selected from: H, alkyl, alkyl substituted with alkylsulfydryl, alkyl substituted with hydroxy, alkyl substituted with sulfydryl, alkyl substituted with -C(=O)NH₂, alkyl substituted with carboxyl, alkyl substituted with amino, alkyl substituted with 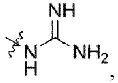 heterocyclylalkyl, and aralkyl;
preferably, R₇ is selected from: H, methyl, isopropyl, sec-butyl, isobutyl, 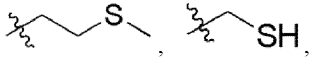 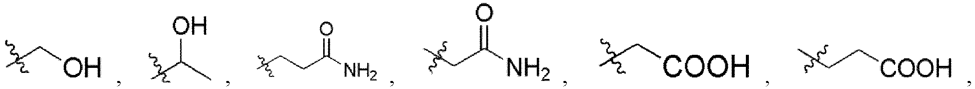 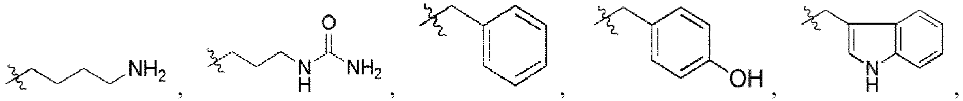 and

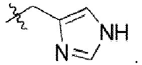

4. The compound according to claim 1, wherein X₅ has the following structure: 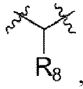 wherein R₈ is selected from: H, alkyl, alkyl substituted with alkylsulfydryl, alkyl substituted with hydroxy, alkyl substituted with sulfydryl, alkyl substituted with -C(=O)NH₂, alkyl substituted with carboxyl, alkyl substituted with amino, alkyl substituted with 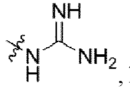 heterocyclylalkyl, and aralkyl; preferably, R₈ is selected from: H, methyl, isopropyl, sec-butyl, isobutyl, 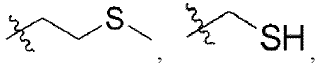 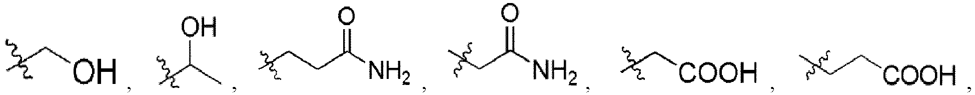 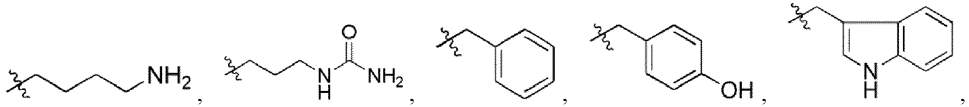 and

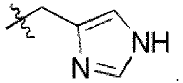

5. The compound according to claim 1, wherein R₂ and R₅ are independently selected from: OH and C1-6 alkoxy;
preferably, R₂ is OH, methoxy, or ethoxy;
preferably, R₅ is OH, methoxy, or ethoxy.

6. The compound according to claim 1, wherein the compound has the following structure:

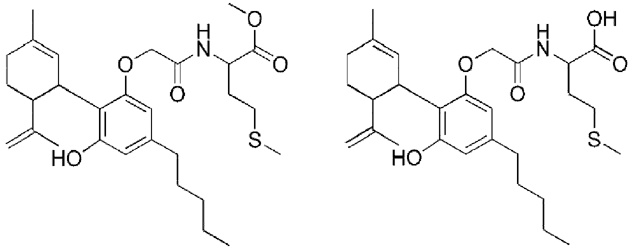

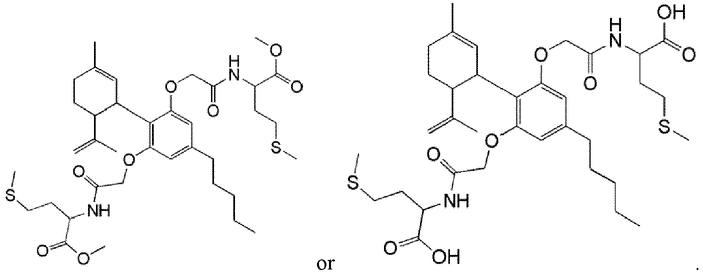

7. The compound according to claim 1, wherein the stereoisomer has the following structure:

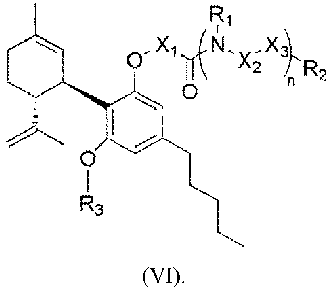

8. The compound according to claim 7, wherein the stereoisomer has the following structure:

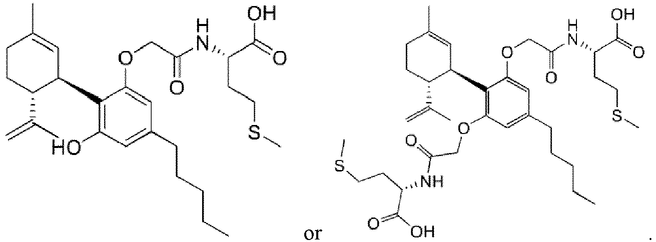

9. A pharmaceutical composition comprising the compound according to any one of claims 1-8, or the pharmaceutically acceptable salt, the stereoisomer, the ester, the prodrug or the solvate thereof, and one or more pharmaceutically acceptable excipients.

10. Use of the compound according to any one of claims 1-8, or the pharmaceutically acceptable salt, the stereoisomer, the ester, the prodrug or the solvate thereof, in the preparation of a medicament for preventing and/or treating a disease, wherein, the disease is selected from: one or more of a nervous system disease, cancer, an autoimmune disease, a cardiovascular disease, pain, inflammation, and liver injury;
preferably, the nervous system disease is selected from: epilepsy, multiple sclerosis, Parkinson's disease, Alzheimer's disease, dementia with Lewy bodies, Huntington's disease, anxiety, and depression;
preferably, the cancer is selected from: breast cancer, lung cancer, colorectal cancer, liver cancer, pancreatic cancer, gastric cancer, gastroesophageal adenocarcinoma, esophageal cancer, small intestine cancer, gastric cardia cancer, endometrial cancer, ovarian cancer, fallopian tube cancer, vulval cancer, testicular cancer, prostate cancer, leukemia, and glioma;
preferably, the autoimmune disease is selected from: systemic lupus erythematosus, rheumatoid arthritis, multiple sclerosis, ulcerative colitis, Crohn's disease, and autoimmune hepatitis.
